# DEMANDE DE BREVET EUROPEEN

(11) **EP 2 926 866 A1**
(43) Date de publication de la demande: **07.10.2015**
(21) Numéro de dépôt: 15161562.2
(22) Date de dépôt: 30.03.2015
(51) Int. Cl.: A61Q 3/02, A61K 8/37

(54) **UTILISATION DE DIBENZOATE DE DIPROPYLÈNEGLYCOL DANS UNE COMPOSITION ANHYDRE APPLICABLE SUR LES ONGLES**

(30) Priorité: 31.03.2014 LU 92415
(71) Demandeur: International Lacquers S.A., 3225 Bettembourg (LU)
(72) Inventeur: Dylewicz, Carole, 57100 Thionville Garche (FR); Armbruster, Marc, 54920 Villers-la-Montagne (FR)
(74) Mandataire: Office Freylinger

(57) **Abrégé**

L'invention concerne l'utilisation de dibenzoate de dipropylèneglycol dans la préparation d'une composition cosmétique anhydre pour vernis à ongles pour améliorer la brillance et/ou pour améliorer l'indice thixotropique de vernis à ongles.

## Description

### Domaine technique

La présente invention concerne des compositions cosmétiques anhydres applicables sur les (faux) ongles et qui forme un film dur, brillant et tenace, ainsi qu'un procédé de préparation.

### Etat de la technique

Les vernis à ongles sont généralement utilisés pour maquiller les ongles ainsi que les faux ongles. Les vernis à ongles sont habituellement composés d'un agent filmogène, d'un solvant, et éventuellement d'un ou plusieurs plastifiants, d'une ou plusieurs résines, d'un ou plusieurs agents de rhéologie et un ou plusieurs colorants.

Les exigences posées aux vernis à ongles sont nombreuses. Les propriétés les plus importantes sont les suivantes :
- Bonne application, avec un pinceau de taille et forme adéquate. Le pinceau permet d'obtenir une surface lisse, sans stries avec une épaisseur constante afin d'obtenir un film et une coloration homogènes.
- Bonnes propriétés de conservation et de stabilité, en particulier une absence totale de sédimentation dans le temps.
- Des temps de séchage et de durcissement courts.
- Une tenue sur l'ongle dans le temps, que l'on définit par la tenue en évitant l'écaillement du vernis ou son usure en bout d'ongle.
- Une brillance importante à l'application, c'est à dire quelques minutes après séchage.
- Une tenue de la brillance dans le temps, c'est à dire une brillance diminuant le moins possible dans les jours qui suivent l'application.

De nouvelles formulations sont continuellement développées pour essayer d'améliorer l'une ou l'autre de ces propriétés.

Parmi ces propriétés, la brillance est particulièrement importante pour l'utilisateur. En effet, pour le consommateur cette caractéristique est un critère significatif puisque la brillance affecte directement l'appréciation esthétique générale de la composition.

La brillance est en principe apportée par différents constituants non volatils du vernis à ongles comme les résines ou la nitrocellulose. Toutefois, ce paramètre est fortement affecté par les composés solides non solubles compris dans la formulation. Parmi ces composés solides non solubles, on citera notamment les pigments qui entraînent par leurs caractéristiques physico-chimiques une diminution de la brillance par rapport à la même formulation non pigmentée. Malgré les différents procédés engagés lors de la préparation de ces pigments, une diminution de la brillance est clairement notée.

Les tentatives d'améliorer la brillance ont été nombreuses et les points d'action sont a priori nombreux. En effet, l'introduction de chacun des ingrédients peut avoir un effet direct ou indirect sur les différentes propriétés du vernis, notamment sur la brillance.

Par conséquent, au vu de la grande variété d'ingrédients utilisés et utilisables dans ce genre de compositions, il existe un réel besoin de mieux maîtriser les résultats obtenus, en particulier en ce qui concerne la brillance.

### Objet de l'invention

Un objet de la présente invention est par conséquent de proposer des formulations du type indiqué ci-dessus, mais présentant une brillance augmentée et de préférence plus durable dans le temps par rapport aux formulations connues.

### Description générale de l'invention

Afin de résoudre le problème mentionné ci-dessus, la présente invention propose l'utilisation de dibenzoate de dipropylèneglycol dans la préparation d'une composition cosmétique anhydre pour vernis à ongles pour améliorer la brillance des vernis à ongles.

En effet, les inventeurs ont découvert d'une manière surprenante que le dibenzoate de dipropylèneglycol permet de réaliser l'objectif d'augmenter nettement la brillance de compositions cosmétiques sans altérer les autres propriétés de manière significative. De plus, cet effet est non seulement immédiat, c'est-à-dire directement obtenu à l'application, mais il est également significativement plus durable dans le temps.

De plus, il a été observé que le dibenzoate de dipropylèneglycol a également un effet bénéfique sur la stabilité des compositions. En effet, de manière imprévue pour l'homme de l'art, le dibenzoate de dipropylèneglycol augmente l'indice thixotropique des solutions dans lesquelles il est ajouté. L'indice thixotropique est une indication sur la rhéologie du milieu et sa capacité à reconstituer le réseau permettant de garantir la stabilité des particules insolubles dans le temps. Il est généralement admis qu'un indice thixotropique élevé est le garant d'une plus grande stabilité dans le temps au niveau du comportement anti-sédimentant et, en même temps d'une bonne fluidité lors de l'application.

En variante ou en plus, la présente invention propose par conséquent aussi l'utilisation de dibenzoate de dipropylèneglycol dans la préparation d'une composition cosmétique anhydre pour vernis à ongles pour améliorer l'indice thixotropique des vernis à ongles.

Il est vrai que le dibenzoate de dipropylèneglycol est connu dans le domaine des vernis à ongles, mais seulement à titre d'agent plastifiant. Ses effets sur la brillance et sur l'indice thixotropique sont tout à fait inattendus.

De préférence, le dibenzoate de dipropylèneglycol sera utilisé en une quantité de 1 à 12 % en poids sec, de préférence 2 à 10 % en poids sec, en particulier de 4 à 8 % en poids sec par rapport au poids total de la composition cosmétique anhydre.

Comme déjà mentionné, le premier avantage de la présente invention consistant à proposer l'utilisation de dibenzoate de dipropylèneglycol est l'accroissement significatif de la brillance de la formulation dès l'application, c'est-à-dire que, contrairement à d'autres agents connus, la brillance est directement améliorée. Le deuxième avantage est que l'effet de brillance est plus durable que pour de nombreux agents connus. Le troisième atout est une amélioration significative de l'indice thixotropique qui a pour conséquences bénéfiques une stabilité accrue de la composition et une bonne fluidité pendant l'application. En conclusion, l'utilisation de dibenzoate de dipropylèneglycol offre le quadruple bénéfice d'une stabilité accrue de la composition permettant une conservation sur de longues périodes sans sédimentation, d'une fluidité accrue pendant l'application, d'un taux de brillance plus important au départ, combiné à un maintien de ce taux élevé de brillance pendant plusieurs jours après application.

D'autre part, l'ajout de dibenzoate de dipropylèneglycol n'affecte pas significativement les autres qualités des compositions, notamment les bonnes caractéristiques d'application et le temps de séchage.

Un avantage additionnel important de la présente invention est que les compositions cosmétiques anhydres, de préférence les formulations de vernis à ongles, à brillance améliorée peuvent comprendre les ingrédients habituellement utilisés par l'homme de l'art. L'homme de l'art n'est donc pas obligé de reconsidérer tous les ingrédients et leurs effets, s'il souhaite utiliser le dibenzoate de dipropylèneglycol comme agent d'amélioration de la brillance. Le dibenzoate de dipropylèneglycol présente en effet une très bonne compatibilité tant avec les solvants, qu'avec les ingrédients courants dans le domaine.

Les compositions cosmétiques anhydres comprennent généralement au moins un solvant organique cosmétiquement acceptable et au moins un agent filmogène.

Les solvants utilisables (seuls ou en combinaison) dans ces compositions sont choisis par exemple parmi les suivants : l'acétate de butyle, l'acétate d'isobutyle, l'acétate de propyle, l'acétate de méthyle, l'acétate d'éthyle, le toluène, l'isopropanol, le diacétone alcool, l'éthanol dénaturé (avec la méthyléthylcétone ou le diéthyléther), le n-butanol, les hydrocarbures linéaires ou cycliques, comme l'hexane, le cyclohexane, l'heptane, l'isododécane. On citera également les dérivés de paraffine de type Isopar qui sont des dérivés paraffiniques qui ont des chaînes carbonées de taille variable, ainsi que les glycols comme l'éthylèneglycol, le propylèneglycol, le dipropylèneglycol ou le tripropylèneglycol.

Les solvants ou leurs mélanges peuvent être utilisés entre 60 et 80 % en poids de la formulation totale. Ils sont sélectionnés selon leurs propriétés physico-chimiques (température d'ébullition par exemple) et leur aptitude à solubiliser les autres constituants de la formulation. Ils seront également sélectionnés selon leurs propriétés organoleptiques (odeur principalement) et leurs possibles utilisations selon les réglementations en vigueur.

De préférence, les compositions comprennent un ou plusieurs autres additifs choisis parmi les agents filmogènes, les résines, les plastifiants, les agents rhéologiques, les absorbants UV, les modificateurs de surface, les agents de soins ou agents dits « traitants », etc.

Le ou les agent(s) filmogène(s) utilisé(s) dans les compositions anhydres pour vernis à ongles peu(ven)t être choisi(s) parmi le groupe constitué par la cellulose, un dérivé de cellulose, une résine vinylique, une résine acrylique et n'importe quelle combinaison de ceux-ci. Les dérivés de cellulose sont par exemple la nitrocellulose, l'acétate butyrate cellulose, l'éthylcellulose et l'hydroxypropylcellulose. Préférentiellement, on choisira la nitrocellulose comme agent filmogène.

Un agent filmogène seul ou une combinaison d'agents filmogènes peut être utilisé(e) dans la composition anhydre pour vernis à ongles dans les proportions allant de 10 % à 20 % en poids sec et de façon préférée de 12 % à 16 % en poids sec par rapport au poids total de la composition.

Les résines pouvant être utilisées sont les suivantes :
- Les résines tosylamide/formaldéhyde ou résines toluènesulfonamide/ formaldéhyde,
- Les résines tosylamide/époxy ou résines toluènesulfonamide/époxy,
- Copolymères acide adipique/néopentyl glycol/anhydride trimellitique,
- Copolymères d'anhydride phtalique/glycérine/glycidyl décanoate,
- Copolymères d'anhydride phtalique/anhydride trimellitique/glycols,
- Copolymères de glycérine/acide phtalique,
- Polymères et copolymères d'acrylates,
- Copolymères d'acrylates et de styrène,
- Copolymères styrène/acrylate/acrylonitrile,
- Sucrose acétate isobutyrate,
- Résine polyvinylbutyral.

De préférence, les compositions ne comprennent pas ou très peu (< 2 %) de composés à base de copolymères d'acrylates, de copolymères d'acrylates et de styrène ou de copolymères styrène/acrylate/acrylonitrile.

En plus de leur action collante, la plupart de ces résines additionnelles ont également une action plastifiante. Les résines additionnelles peuvent être utilisées dans la formule, seules ou sous la forme de mélanges, dans des quantités allant de 3 à 20 % en poids sec, de préférence 5 à 12% en poids sec, en particulier de 7 à 10 % en poids sec par rapport au poids total de la composition.

Les compositions anhydres pour vernis à ongles peuvent également comprendre des plastifiants (autres que le dibenzoate de dipropylèneglycol). Les plastifiants sont des composés ayant une température d'ébullition élevée qui ne s'évaporent pas lors du séchage du vernis à ongles. Ils sont utilisés pour moduler la dureté du film formé afin d'obtenir les caractéristiques physico-chimiques du film souhaitées et représentent en général de 2 à 12 % en poids sec, de préférence 5 à 10 % en poids sec, en particulier de 6 à 8 % en poids sec par rapport au poids total de la composition.

Des exemples de plastifiants utilisables dans l'invention sont l'acétyl tributyl citrate, l'acétyl triéthyl citrate, le tributyl citrate, le triéthylcitrate, le dibutyl phtalate, le triphénylphosphate, la triacétine, le triméthyl pentanyl diisobutyrate, la triéthylhexanoïne, le sucrose benzoate, le dibutyl adipate, le diéthyl phthalate, le diisobutyl adipate, le diisopropyl adipate, etc.

Pour colorer ou fournir des effets particuliers à la composition anhydre pour vernis à ongles, elle peut comprendre un ou plusieurs agents de coloration choisis parmi les pigments, les nacres, les glitters (paillettes) et/ou les particules métalliques. Ces agents de coloration fournissent l'aspect esthétique recherché par les utilisateurs de vernis à ongles. Ces agents de coloration peuvent produire des effets très différents tels que « irisé », « métallisé », « nacré », « miroir », « crème », « pailleté », craquelé », « mat », « néon », « fluorescent », « holographique », etc.

Par exemple, les compositions cosmétiques pourront comprendre de 0.1 à 20 % en poids et de préférence 0.5 à 12 % en poids sec par rapport au poids total de la composition d'une ou de plusieurs matières colorantes ou agents de coloration, comme les pigments et les particules nacrantes (nacres), les glitters (paillettes) ou les particules métalliques d'aluminium ou autres.

En effet, comme mentionné, les inventeurs ont découvert que l'utilisation de dibenzoate de dipropylèneglycol dans une composition cosmétique anhydre comme par exemple une formulation de vernis à ongles ne permettait pas seulement d'en améliorer la brillance, notamment en réduisant l'effet négatif sur la brillance surtout des composants colorants comme les pigments, mais également d'augmenter la résistance à la sédimentation par accroissement de l'indice thixotropique. Ce dernier effet est évidemment encore plus important lorsque les compositions comprennent des particules en suspension, comme les pigments, des nacres, des paillettes, des particules métalliques, etc. Dans le cadre de la présente invention, le dibenzoate de dipropylèneglycol peut être un mélange d'isomères, mais de préférence le dibenzoate de dipropylèneglycol utilisé est celui avec le numéro CAS 27138-31-4, de manière particulièrement préférée il répond à la formule [C₆H₅C(O)OCH(CH₃)CH₂]₂O.

Parmi les pigments utilisables dans l'invention on citera le dioxyde de titane (Cl 77891), l'oxyde de fer noir (Cl 77499), l'oxyde de fer rouge (Cl 77491), le DC Red 6 Ba Lake (Cl 15850), le DC Red 7 Ca Lake (Cl 15850:1), le DC Red 34 Ca Lake (Cl 15880), le FDC Yellow 5 Al Lake (Cl 19140), le FDC Blue 1 Al Lake (Cl 42090), le bleu ultramarine (Cl 77007), le DC Violet 2 (Cl 60725), le DC Black 2 (Cl 77266), etc.

Les nacres sont également utilisables dans l'invention, les nacres sont des particules minérales de différentes natures recouvertes d'une ou plusieurs couches d'oxydes (oxydes de titane ou oxydes de fer) ou encore de pigments. Les nacres peuvent se présenter sous la forme d'une poudre ou de plaque de tailles variées. Parmi les nacres utilisables dans l'invention, on peut citer :
- les nacres préparées à partir de mica naturel et ayant subi des opérations de coating successives,
- les nacres préparées à partir de mica synthétique appelé également « synthetic fluorphlogopite »,
- les nacres préparées à partir de calcium sodium borosilicate,
- les nacres préparées à partir d'oxyde de silice.

Les glitters peuvent être préparés à partir de plusieurs types de matériaux comme les films polyesters comportant en surface une couche métallique, le polyéthylène téréphtalate, le polybutylène téréphtalate, les polyuréthanes, les copolymères d'acrylates, les copolymères d'éthylène et acétate de vinyle ou encore l'aluminium.

Différents autres additifs sont utilisables dans les compositions sont les suivants.

Parmi les additifs de rhéologie, on citera notamment les dérivés d'argile modifiés comme le stéaralkonium hectorite ou le stéaralkonium bentonite. Ces additifs de rhéologie seront de préférence utilisés de 0.1 à 3 % en poids sec par rapport au poids total de la formulation. Ces additifs permettent notamment la suspension des particules insolubles dans le vernis tels que les pigments ou encore les nacres.

Parmi les absorbants UV, on citera les absorbants de lumière UV qui permettent de protéger la formulation ou les agents colorants utilisés l'invention des rayonnements UV. Parmi les protecteurs UV utilisables dans l'invention, on citera : la benzophénone-1 (CAS 131-56-6), la benzophénone-3 (CAS 131-57-7), le benzyl salicylate (CAS 118-58-1), l'étocrylène (CAS 5232-99-5), le drométrizole (CAS 2440-22-4), le butyl méthoxydibenzoylméthane (CAS 70356-09-1), etc.

Les absorbants UV seront utilisés seuls ou sous la forme de combinaison dont le pourcentage total dans la formule peut aller de 0.1 à 1% en poids sec par rapport au poids total de la composition.

Parmi les additifs utilisables selon l'invention, on citera également les modificateurs de surface comme les cyclométhicones ou cyclopentasiloxane, les diméthicones et le triméthylsiloxysilicate.

Ces additifs de surface sont généralement utilisés dans des faibles quantités dans la formulation allant de 0.01 à 2 % en poids sec par rapport au poids total de la formulation.

On citera également les additifs dits « traitants » de l'ongle utilisables dans la composition selon l'invention. Les additifs traitants peuvent représenter de 0.01% à 5% en poids sec, de préférence de 0.05% à 2% en poids sec par rapport au poids total de la composition totale. Parmi ces additifs « traitants », on citera :
- le formaldéhyde utilisé comme durcisseur de l'ongle,
- la vitamine E acétate (tocophéryl acétate),
- la vitamine A palmitate (rétinyl palmitate),
- les dérivés organiques ou inorganiques de calcium tels que le chlorure de calcium, le pantothénate de calcium,
- le diméthyloxobenzodioxasilane,
- la myrrhe,
- les acides aminés soufrés comme la cystéine, ses sels et leurs dérivés, la cystine, le glutathion. Les acides aminés soufrés sont en effet capables de créer des cross-linkings entre la kératine et les groupes SH libres de ces dérivés soufrés,
- la kératine hydrolysée d'origine végétale comme le Prosina de Croda,
- les alpha-hydroxyacides comme l'acide citrique, l'acide ascorbique,
- la biotine,
- l'urée et la diméthylurée.

L'invention concerne également un procédé de préparation d'une composition cosmétique anhydre, notamment pour vernis à ongles ou de soins des ongles, à brillance améliorée, dans lequel on mélange au moins un solvant organique cosmétiquement acceptable, un agent filmogène et du dibenzoate de dipropylèneglycol. De préférence, on ajoutera à la composition au moins un agent de coloration choisi parmi les pigments, les nacres, les glitters et/ou les particules métalliques, ainsi qu'éventuellement un ou plusieurs autres additifs choisis parmi les résines, les plastifiants autres que le dibenzoate de dipropylèneglycol, les agents rhéologiques, les absorbants UV et les modificateurs de surface, les agents dits « traitants », etc.

Un avantage supplémentaire de l'invention est que le dibenzoate de dipropylèneglycol en tant qu'agent d'amélioration de la brillance peut non seulement être ajouté à n'importe quel moment du procédé, c'est-à-dire que son effet n'est pas affecté par l'ordre dans lequel les ingrédients sont dosés. De plus, en complément des avantages déjà mentionnés ci-dessus, il est à remarquer que le procédé ci-dessus peut être réalisé moyennant des installations tout à fait standard et ne demande pas de connaissances, ni de précautions particulières de l'opérateur.

Tous les modes de réalisation cités précédemment peuvent être combinés sous réserve de leur faisabilité technique.

### Exemples

Les exemples, ci-dessous, concernent diverses formulations de compositions anhydres pour vernis à ongles.

Essais 1 à 4 comparés à une base standard

Cinq formulations distinctes ont été évaluées au niveau de leur propriétés de brillance d'une part au moyen d'un brillancemètre à un angle de 60° et d'autre part par un panel d'utilisateurs. Le panel d'utilisateurs a attribué une note à chacune de ces formulations :
- 1 étant la note la plus faible et correspondant à un résultat médiocre,
- 5 étant la note la plus élevée et correspondant à un résultat excellent.
Les utilisateurs ont appliqué chaque composition sur leurs ongles afin d'en estimer leurs propriétés.

L'impact sur les autres propriétés sensorielles a également été vérifié.

La composition correspondant à la base dite standard ne comprend pas de dibenzoate de dipropylèneglycol. Les compositions correspondant aux essais 1-4 comprennent diverses concentrations de dibenzoate de dipropylèneglycol en remplacement (partiel ou total) du plastifiant courant acétylcitrate de tributyle (nom INCI : acetyl tributyl citrate).

**Tableau 1**

| **Ingrédients** | **Fonction** | **Base standard** | **Essai 1** | **Essai 2** | **Essai 3** | **Essai 4** |
|---|---|---|---|---|---|---|
| Acétate d'Ethyle | Solvant | 35,5 | 35,5 | 35,5 | 35,5 | 35,5 |
| Acétate de Butyle | Solvant | 28,5 | 28,5 | 28,5 | 28,5 | 28,5 |
| Alcool iso-propylique (provenant de la Nitrocellulose) | Solvant | 5,6 | 5,6 | 5,6 | 5,6 | 5,6 |
| Acétyl Tri-Butyl Citrate | Plastifiant | 6,5 | 4,875 | 3,25 | 1,625 | 0 |
| Dibenzoate de dipropylèneglycol | Plastifiant | **0** | **1,625** | **3,25** | **4,875** | **6,5** |
| Nitrocellulose (en poids sec) | Filmogène | 13,2 | 13,2 | 13,2 | 13,2 | 13,2 |
| Phthalic Anhydride / Trimellitic Anhydride / Glycols copolymer (en poids sec) | Résine | 9,1 | 9,1 | 9,1 | 9,1 | 9,1 |
| Bentonite | Agent thixotropant | 1,6 | 1,6 | 1,6 | 1,6 | 1,6 |
| **Total (% en poids)** | | 100 | 100 | 100 | 100 | 100 |
| **Brillance sur carte mesurée par Brillancemètre** | | | | | | |
| Brillance à 60 ° | % | 84 | 85 | 86 | 87 | 88 |
| **Propriétés sensorielles évaluées par le panel d'utilisateurs** Notation sensorielle : 1 = médiocre / 5 = excellent | | | | | | |
| Brillance sur ongle | | 3,00 | 3,50 | 3.75 | 4,00 | 4,25 |

Les essais montrent que l'utilisation de dibenzoate de dipropylèneglycol dans la composition affecte positivement la brillance, tant objectivement (brillancemètre) que subjectivement (panel d'utilisateurs).

De plus, il est remarquable que cette augmentation de la brillance n'a pas impactée négativement les autres propriétés sensorielles par le dibenzoate de dipropylèneglycol, en l'occurrence le temps de séchage, le temps de durcissement, la couvrance, l'application, l'étalement et l'homogénéité.

Brillance en fonction de la concentration

Six autres compositions ont été évaluées avec des concentrations croissantes d'un plastifiant courant (Acétyl Tri-Butyl Citrate) et de dibenzoate de dipropylèneglycol selon l'invention.

**Tableau 2 : Effet sur la brillance**

| | **4%** | **6%** | **10%** |
|---|---|---|---|
| Acétyl Tri-Butyl Citrate | 84 | 84 | 84 |
| Dibenzoate de dipropylèneglycol | 86 | 88 | 91 |

La brillance (Brillance à 60 °) augmente avec la concentration de dibenzoate de dipropylèneglycol, ce qui n'est pas le cas avec le plastifiant classique (Acétyl Tri-Butyl Citrate).

Indice thixotropique en fonction de la concentration

Huit compositions supplémentaires ont été analysées avec des concentrations croissantes d'un plastifiant courant (Acétyl Tri-Butyl Citrate) et de dibenzoate de dipropylèneglycol selon l'invention.

**Tableau 3 : Effet sur l'indice thixotropique**

| | **4%** | **6%** | **8%** | **10%** |
|---|---|---|---|---|
| Acétyl Tri-Butyl Citrate | 1,6 | 1,6 | 1,5 | 1,4 |
| Dipropylene Glycol Dibenzoate | 1,65 | 1,7 | 1,75 | 1,8 |

Les hautes concentrations en acétyl tri-butyl citrate se font au détriment de la thixotropie qui diminue au-delà de 6 %. Au contraire, le dibenzoate de dipropylèneglycol améliore l'indice thixotropique avec sa concentration.

L'indice thixotropique est le rapport entre Viscosité V3 et Viscosité V2 mesurées sur un viscosimètre Brookfield
V2 : viscosité (mPa.s) mesurée à 60 rpm
V3 : viscosité mesurée à 6 rpm

## Revendications

1. Utilisation de dibenzoate de dipropylèneglycol dans la préparation d'une composition cosmétique anhydre pour vernis à ongles pour améliorer la brillance et/ou pour améliorer l'indice thixotropique des vernis à ongles.

2. Utilisation selon la revendication 1, dans laquelle la quantité de dibenzoate de dipropylèneglycol représente 1 à 12 % en poids sec, de préférence 2 à 10 % en poids sec, en particulier de 4 à 8 % en poids sec par rapport au poids total de la composition cosmétique anhydre.

3. Utilisation selon l'une des revendications 1 ou 2, dans laquelle la composition cosmétique anhydre comprend en outre au moins un solvant organique cosmétiquement acceptable et au moins un agent filmogène.

4. Utilisation selon l'une quelconque des revendications précédentes, dans laquelle la composition cosmétique anhydre comprend en outre de 0.1 à 20 % en poids sec par rapport au poids total de la composition d'un ou de plusieurs agents de coloration choisis parmi les pigments, les nacres, les glitters et/ou les particules métalliques.

5. Utilisation selon l'une quelconque des revendications précédentes, dans laquelle la composition cosmétique anhydre comprend en outre un ou plusieurs autres additifs choisis parmi les agents filmogènes, les résines, les plastifiants autre que le dibenzoate de dipropylèneglycol, les agents rhéologiques, les absorbants UV, les modificateurs de surface et les agents dits « traitants ».

6. Procédé de préparation d'une composition cosmétique anhydre pour vernis à ongles selon l'une quelconque des revendications 3 à 5, comprenant le mélange d'au moins un solvant organique cosmétiquement acceptable et du dibenzoate de dipropylèneglycol.

7. Procédé selon la revendication 6, dans lequel la quantité de dibenzoate de dipropylèneglycol représente 1 à 12 % en poids sec, de préférence 2 à 10 % en poids sec, en particulier de 4 à 8 % en poids sec par rapport au poids total de la composition cosmétique anhydre.

8. Procédé selon la revendication 6 ou 7, comprenant en outre l'incorporation dans le mélange d'au moins un agent de coloration choisi parmi les pigments, les nacres les glitters et/ou les particules métalliques.

9. Procédé selon l'une quelconque des revendications 6 à 8, comprenant en outre l'incorporation dans le mélange d'un ou de plusieurs autres additifs choisis parmi les agents filmogènes, les résines, les plastifiants, les agents rhéologiques, les absorbants UV, les modificateurs de surface et les agents dits « traitants ».
